# FASCICULE DE BREVET EUROPEEN

(11) **EP 2 827 840 B1**
(45) Date de publication et mention de la délivrance du brevet: **28.08.2019**
(21) Numéro de dépôt: 13715338.3
(22) Date de dépôt: 15.03.2013
(51) Int. Cl.: A61K 38/13, A61K 9/00, A61K 9/08, A61K 47/32, A61K 47/44, A61K 47/38, A61P 27/02

(54) **SOLUTION OPHTALMIQUE AQUEUSE A BASE DE CICLOSPORINE A**
CYCLOSPORIN A-BASIERTE WÄSSRIGE OPTHALMISCHE LÖSUNG
CYCLOSPORIN A-BASED AQUEOUS OPHTHALMIC SOLUTION

(30) Priorité: 22.03.2012 FR 1252583; 22.03.2012 US 201261614218 P
(43) Date de publication de la demande: 28.01.2015
(73) Titulaire: Laboratoires THEA, 63100 Clermont-Ferrand (FR)
(72) Inventeur: MURIAUX, Emmanuel, F-78580 Maule (FR); MERCIER, Fabrice, F-63000 Clermont-ferrand (FR)
(74) Mandataire: Cabinet Laurent & Charras
(86) Numéro de dépôt international: PCT/FR2013/050557
(87) Numéro de publication internationale: WO 2013/140071

(56) Documents cités:
- EP-A1- 1 142 566
- WO-A1-93/23010
- WO-A1-2009/046967
- WO-A1-2009/088570
- WO-A2-2009/058585
- WO-A2-2009/099467
- US-A1- 2002 173 516
- LALLEMAND F ET AL: "Cyclosporine A delivery to the eye: A pharmaceutical challenge", EUROPEAN JOURNAL OF PHARMACEUTICS AND BIOPHARMACEUTICS, ELSEVIER SCIENCE PUBLISHERS B.V., AMSTERDAM, NL, vol. 56, no. 3, 1 novembre 2003 (2003-11-01), pages 307-318, XP004470466, ISSN: 0939-6411, DOI: 10.1016/S0939-6411(03)00138-3

## Description

### DOMAINE DE L'INVENTION

La présente invention propose une solution ophtalmique aqueuse contenant comme actif un agent immunosuppresseur, à savoir la ciclosporine A. Ladite solution présente des caractéristiques toxicologiques et allergéniques acceptables pour le traitement des troubles de la surface oculaire ayant une composante immunitaire inflammatoire, tels que la sécheresse oculaire ou la perte de sensibilité de la cornée.

Plus précisément, l'invention a pour objet une solution ophtalmique aqueuse dans laquelle l'actif, la ciclosporine A, est formulé à l'aide d'un système polymérique ternaire permettant sa solubilisation dans une quantité thérapeutiquement efficace et une grande stabilité, et ce même en l'absence de tout conservateur.

### ETAT DE LA TECHNIQUE

Le syndrome de l'oeil sec, également dénommé sécheresse oculaire ou kératoconjonctivite sèche (KCS), est une pathologie pour laquelle la quantité de larmes produites par les glandes lacrymales est insuffisante. Il en résulte un inconfort au niveau de l'oeil, qui s'accompagne de démangeaisons, picotements et/ou sensations de brûlures.

La sécheresse oculaire peut résulter d'une anomalie des glandes lacrymales, d'une inflammation des paupières, d'une inflammation oculaire due à une allergie, d'une chirurgie réfractive (notamment au laser), d'une carence en certains lipides dans l'alimentation quotidienne, d'un port prolongé de lentilles de contact, d'une altération hormonale, d'une maladie auto-immune, ou d'effets secondaires de certains médicaments.

La perte de sensibilité de la cornée, quant à elle, peut résulter par exemple des suites d'une chirurgie, d'une ulcération ou d'une infection virale (kératite virale) de la cornée.

Dans le traitement de la sécheresse oculaire, l'application de larmes artificielles, également appelées gouttes ophtalmiques lubrifiantes ou humectantes, apporte un soulagement local mais de courte durée, sans solutionner le problème d'un point de vue systémique : elles n'aident en rien l'organisme à améliorer la quantité ou la qualité des larmes et ne constituent qu'une solution de substitution temporaire. Les larmes artificielles de viscosité faible à moyenne sont généralement à base d'alcools polyvinyliques ou de dérivés cellulosiques, alors que celles à viscosité plus élevée contiennent des carbomères ou de l'acide hyaluronique.

Un traitement plus prometteur de ces deux type de pathologies repose sur l'application topique de ciclosporine A (ou cyclosporine A).

La ciclosporine A, couramment appelée ciclosporine, est un agent immunosuppresseur efficace, notamment utilisé dans le domaine de la transplantation d'organes et pour prévenir le rejet aigu des allogreffes.

Du point de vue de son mode d'action, il semble admis que la ciclosporine inhibe la calcineurine, une phosphatase impliquée dans la transcription du gène de l'interleukine 2, normalement sécrétée par les lymphocytes T. En outre, la ciclosporine A inhibe la production de lymphokines et la libération d'interleukines, ce qui conduit à une diminution substantielle de l'activité des lymphocytes T effecteurs. En d'autres termes, la ciclosporine A est une molécule apte à inhiber ou prévenir l'activité du système immunitaire.

La ciclosporine A est un peptide cyclique de onze acides aminés, synthétisé par un champignon microscopique *Tolypocladium inflatum*. La ciclosporine A a pour formule [R-[[R*,R*-(E)]]-cyclic(L-alanyl- D-alanyl-N-methyl-L-leucyl- N-methyl-L-leucyl-N-methyl- L-valyl-3-hydroxy-N,4-dimethyl- L-2-amino-6-octenoyl-L-a-amino- butyryl-N-methylglycyl-N-methyl- L-leucyl-L-valyl-N-methyl-L-leucyl) (numéro CAS 59865-13-3), et pour structure :

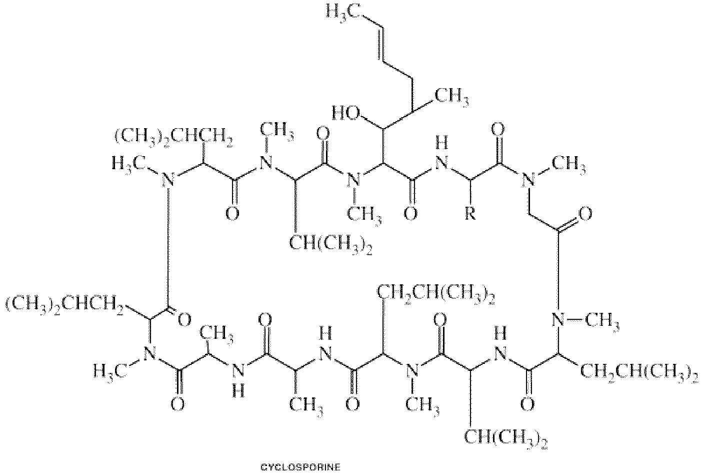

Une difficulté pratique liée à la ciclosporine A est qu'elle présente une très faible solubilité en milieux aqueux, de l'ordre de 20-30 µg/l à 25°C soit 0.002 à 0.003% en poids (w/v). En revanche, cette molécule est soluble dans l'alcool (par exemple éthanol ou méthanol), l'acétonitrile, l'acétate d'éthyl et les huiles (par exemple huile d'olive, huile de mais ou huile de ricin).

A noter que ce même problème de solubilité se pose pour d'autres agents immunosuppresseurs ophtalmiques tels que le tracolimus (Numéro CAS : 104987-11-3) ou la rapamycine (Numéro CAS : 53123-88-9).

Des efforts importants ont été réalisés pour obtenir une formulation de collyre pour le traitement oculaire de la sécheresse oculaire et la perte de la sensibilité de la cornée, pour laquelle la tolérance à l'instillation et la biodisponibilité de la ciclosporine A dans les tissus ciblés sont acceptables.

Ainsi, la tolérance se mesure par l'irritation du collyre provoqué dans l'oeil après son instillation, par exemple à l'aide de l'échelle établie par Draize et al. (Methods for the study of irritation and toxicity of substances applied topically to the skin and mucous membranes. J. Pharmacol. And Exp. Therapeutics, 1944; 82: 377-390).

Par ailleurs, l'efficacité de pénétration du collyre reflète la biodisponibilité de la ciclosporine A dans les différents tissus rencontrés après instillation, à savoir les conjonctives (bulbaires et palpébrales) et la cornée.

Etant donné la très faible solubilité de la ciclosporine A dans l'eau, les développements dans le domaine ophtalmique se sont focalisés sur des collyres sous forme d'émulsions ou de solutions hydro-alcooliques.

Ainsi, la ciclosporine A est commercialement disponible aux Etats-Unis sous la forme d'une émulsion à 0.05% dans l'huile de ricin. Ce produit, vendu sous forme d'unidoses sans conservateur sous le nom Restasis™, est préconisé pour le traitement de la kératoconjonctivite sèche.

En Europe, et plus particulièrement en France, l'administration de ciclosporine A repose essentiellement sur des préparations hospitalières. Toutefois, ces préparations hospitalières, dont les concentrations varient entre 0.05 et 2% en actif formulé en milieux huileux (huile d'olive, huile de ricin ou huile de maïs) et/ou en présence d'alcool, ne sont stables que pendant quelques semaines.

En outre et dans les deux cas, la tolérance à l'instillation n'est pas bonne, ce qui nuit à la bonne observance du traitement et donc à son efficacité.

En effet, Nourry et al. (Etude de la cytotoxicité de différents collyres à base de ciclosporine A buvable (Sandimmun™). J. Fr. Ophtalmol., 2006 ; 29, 3, 251-257) ont suggéré que les excipients (huile et/ou alcool) de ces collyres provoquent des effets secondaires, comme l'irritation des conjonctives ou une hypérémie, une toxicité de l'épithélium cornéen, des démangeaisons, ou des sensations de brûlures. Ces mêmes auteurs ont comparé la tolérance de collyres hydro-alcooliques et huileux (émulsions) et concluent que les collyres huileux sont moins irritants que les collyres hydro-alcooliques à l'instillation.

C'est pourquoi, les développements les plus récents, dans le domaine des collyres à base notamment de ciclosporine A, visent à améliorer l'efficacité des collyres huileux, ou émulsions, afin d'augmenter leur tolérance à l'instillation, tout en contournant les problèmes de solubilité.

Il existe toutefois un besoin évident de développer des formulations ophtalmiques à base d'immunosuppresseur, en particulier de ciclosporine A, présentant les caractéristiques suivantes :
- se présentant sous la forme de solutions aqueuses,
- stables dans le temps,
- bien tolérées par l'oeil après instillation,
- possédant une bonne pénétration dans la cornée,
- permettant d'incorporer des doses adaptées pour le traitement notamment du syndrome de l'oeil sec et de la perte de la sensibilité de la cornée.

Le document WO2009/099467 décrit des compositions à base de ciclosporine A, éventuellement sous forme de solutions formulées à l'aide de 2 polymères. Le document US2002/173516 décrit des compositions à base de tacrolimus, éventuellement sous forme de solutions formulées à l'aide de 2 polymères.

### DESCRIPTION DE L'INVENTION

Dans le cadre de la présente invention et malgré le caractère peu soluble des immunosuppresseurs ophtalmiques, le Demandeur a développé une solution aqueuse stable, possiblement dépourvue de conservateur, dont la concentration en ciclosporine A garantit l'efficacité du produit et dont la formulation galénique garantit une très bonne tolérance à l'instillation.

Ainsi et selon un premier aspect, la présente invention concerne une solution ophtalmique aqueuse comprenant :
- la ciclosporine A en tant qu'agent immunosuppresseur;
- un dérivé cellulosique en tant que premier polymère ;
- un dérivé polyvinylique en tant que deuxième polymère ;
- un hydroxystéarate de macrogolglycérol en tant que troisième polymère.

L'utilisation combinée de 3 polymères, judicieusement choisis, permet ainsi de solubiliser dans l'eau une quantité thérapeutiquement efficace de l'immunosuppresseur, à savoir la ciclosporine A, tout en conférant à ladite composition une viscosité adaptée à l'application topique oculaire. En d'autres termes, les 3 polymères en présence jouent un rôle dans la solubilisation et/ou la gélification et sont définis comme des agents solubilisant et/ou gélifiant, voire co-solubilisant et/ou co-gélifiant.

La solution ophtalmique aqueuse selon l'invention comprend donc, comme principe actif, un agent immunosuppresseur peu soluble, la ciclosporine A. Il est toutefois décrit la possibilité d'appliquer ce même principe de formulation à d'autres agents immunosuppresseurs topiques oculaires présentant une faible solubilité dans l'eau tels que les autres formes de la ciclosporine, le tracolimus (Numéro CAS : 104987-11-3) ou la rapamycine (Numéro CAS : 53123-88-9).

De manière adaptée, la concentration en actif, à savoir en ciclosporine A, dans la solution est comprise entre 0,01 et 0,2 % en poids de la solution (poids/volume ou w/v), avantageusement entre 0,05 et 0,1%. Il s'agit de quantités à la fois thérapeutiquement efficaces et compatibles avec les limites de solubilisation du système de formulation proposé. Selon un mode de réalisation privilégié, la concentration est supérieure ou égale à 0,05% en poids, voire supérieure à 0,05%, voire même supérieure ou égale à 0,1% en poids, ce qui correspond à une quantité solubilisée jamais atteinte dans le contexte d'une solution aqueuse. Pour d'autres agents immunosuppresseurs que la ciclosporine, notamment ceux présentant une solubilité dans l'eau supérieure, la proportion poids/volume de cet agent dans la solution selon l'invention peut être supérieure ou égale à 0,1%, voire supérieure ou égale à 0,2%, voire même supérieure ou égale à 0,5% ou même 1%.

A noter qu'une solution selon l'invention peut également comprendre d'autres actifs de la même classe ou d'une autre classe. De manière adaptée, le ou les autres actifs se présentent également sous une forme solubilisée. De manière avantageuse, il ne s'agit pas d'un corticostéroïde.

De manière caractéristique, la composition selon l'invention se présente sous la forme d'une solution aqueuse. Par définition, une solution aqueuse est une phase liquide contenant plusieurs espèces chimiques, dont une ultra-majoritaire à savoir l'eau (H₂O) qui sert de solvant (ou véhicule) aux espèces ultra-minoritaires constituant les solutés ou « espèces chimiques dissoutes ». De manière avantageuse, la solution selon l'invention ne contient pas de solvant organique, en particulier pas d'alcool tel que l'éthanol.

Dans le cadre de l'invention, la ciclosporine se trouve donc dissoute dans la solution, et non sous forme de micelles ou en émulsion (dans une phase huileuse) comme dans l'art antérieur.

De manière avantageuse, la solution selon l'invention est donc dépourvue d'huile, notamment d'origine végétale telle que l'huile de ricin.

Selon un autre mode de réalisation privilégiée, la solution selon l'invention est essentiellement dépourvue de particules, notamment selon la méthode 2.9.19 de la Pharmacopée Européenne. Notamment, la solution est avantageusement dépourvue de micelles.

Selon l'invention, cette solubilisation est assurée par un système ternaire de polymères :
La solution ophtalmique aqueuse selon l'invention comprend un premier polymère choisi parmi les dérivés cellulosiques, plus précisément les éthers de cellulose. De manière surprenante, alors que ces dérivés sont connus pour leurs propriétés gélifiantes, le Demandeur a observé qu'il permettait une pré-solubilisation de l'immunosuppresseur, à savoir de la ciclosporine A. Ce premier polymère peut donc être défini comme un agent co-solubilisant/co-gélifiant.

De manière avantageuse, le dérivé de la cellulose est choisi dans le groupe comprenant : la méthylcellulose, l'éthylcellulose, l'hydroxyéthylcellulose, l'hydroxypropylcellulose, la carboxyméthylcellulose, l'hydroxypropylméthylcellulose, ou leurs sels. Encore plus avantageusement, il s'agit de la carboxyméthylcellulose de sodium (Na).

De manière adaptée, la concentration de ce premier polymère, à savoir un dérivé cellulosique, est comprise entre 0,1 et 3% en poids de la solution (poids/volume ou w/v), avantageusement entre 0,5 et 1,5%, encore plus avantageusement égale à 0.8%.

Selon un mode de réalisation privilégié, l'agent immunosuppresseur, à savoir la ciclosporine A, est pré-solubilisée dans une première étape en présence de ce premier polymère dans l'eau. Cette première étape aboutit à une suspension, laquelle sera ensuite mélangée aux deux autres polymères de la solution.

La solution ophtalmique aqueuse selon l'invention comprend un deuxième polymère de type polyvinylique, avantageusement de l'alcool polyvinylique.

L'alcool polyvinylique (Numéro CAS 9002-89-5) est connu pour ses propriétés gélifiantes, d'où son utilisation en tant qu'agent gélifiant. De manière remarquable, ce dérivé polyvinylique participe à la co-solubilisation de la ciclosporine A, potentiellement à l'aide des groupements hydroxyle.

Un autre dérivé polyvinylique qui peut être utilisé est le polyvinyl pyrrolidone ou povidone (PVP) (CAS n° 9003-39-8).

De manière adaptée, la concentration du second polymère, à savoir un dérivé polyvinylique, est comprise entre 0,25 et 3% en poids de la solution (poids/volume ou w/v), avantageusement égale à 0,5%.

De manière remarquable, ces 2 catégories de polymères sont choisies parmi la famille des excipients largement utilisés dans le cadre de la formulation de collyres destinés au traitement de sécheresse oculaire. Ainsi, alors que dans le cadre du traitement par le Restasis™, il est préconisé l'application concomitante de larmes artificielles, la solution ophtalmique selon l'invention constitue un produit « 2 en 1 ».

De manière caractéristique, la solution ophtalmique aqueuse selon l'invention comprend un troisième polymère jouant le rôle d'agent solubilisant majeur, avantageusement un polymère non ionique. Il s'agit avantageusement d'un polymère porteur d'une fonction lipophile, à savoir un hydroxystéarate de macrogolglycérol. L'hydroxystéarate de macrogolglycérol est un agent solubilisant non ionique, apte à co-solubiliser par exemple la ciclosporine A, grâce notamment à sa partie apolaire.

Ces polymères sont composés d'une molécule d'acide gras en C18, oméga-9 et hydroxylé, l'acide ricinoléique ou acide (*R*)-(+)-12-hydroxy-9Z-octadécénoïque (partie apolaire) et de 35 molécules de polyéthylène glycol H-(0-CH=CH)ₙ-OH, à chaîne carbonée de longueur variable (C₇, C₂₅, C₄₀ ou C₆₀) définissant les grades 7, 25, 40 ou 60.

Encore plus avantageusement, le troisième polymère est l'hydroxystéarate de macrogolglycérol 40, également appelé PEG 40 hydrogenated castor oil, possédant le numéro CAS 61788-85-0.

De manière adaptée, la concentration du troisième polymère, à savoir l'hydroxystéarate de macrogolglycérol, est comprise entre 0,5 et 20% en poids de la solution (poids/volume ou w/v), avantageusement entre 5 et 15%, encore plus avantageusement égale à 10%. Il constitue donc le polymère majoritaire du système ternaire.

De manière avantageuse, la solution selon l'invention ne comprend pas de cyclodextrines. Selon un autre mode de réalisation, elle ne comprend pas de composés appelés « penetration enhancer » tels que le BAK ou le DMSO qui sont connus pour aider à la solubilisation des actifs et déstabiliser les membranes des cellules constituant l'épithélium cornéen.

De manière avantageuse, l'association de ces trois polymères confère à la solution selon l'invention une viscosité adaptée à l'application topique ophtalmique visée.

Ainsi et de manière adaptée, la solution selon l'invention présente une viscosité inférieure ou égale à 50 mPa.s (cP) avantageusement comprise entre 5 et 50 mPa.s, et encore plus avantageusement comprise entre 5 et 15 mPa.s, lorsqu'elle est mesurée à l'aide d'un viscosimètre à mobile tournant BROOKFIELD RVDV III à 25°C.

En pratique, cette viscosité est avantageusement proche de la viscosité des larmes humaines, évaluée à environ 6,5 mPa.s à 20°C et à gradient de vitesse proche de zéro. L'application de la solution selon l'invention ne provoque ainsi aucune sensation de gêne ou d'irritation. En effet, il a été déterminé qu'une élimination aisée et non gênante n'est possible que si la viscosité du mélange solution ophtalmique / larme est inférieure à 50 mPa.s.

Bien entendu, la solution ophtalmique aqueuse peut contenir en outre un additif choisi dans le groupe des agents isotonisants de type non ioniques, des agents antioxydants et/ou des systèmes tampons.

De manière préférée, la solution selon l'invention présente un pH neutre, avantageusement compris entre 6,5 et 7. Préférentiellement, elle correspond à une formulation isotonique, avec une osmolalité avantageusement comprise entre 290 et 350 mosmol/kg.

Selon un mode de réalisation particulier, la solution selon l'invention est constituée des ingrédients listés dans le tableau ci-dessous, avantageusement selon la formule centésimale indiquée :

| **PRODUIT** | **FONCTION** | **FORMULE CENTESIMALE (g/100g)** |
|---|---|---|
| Ciclosporine A | Produit actif | 0.01 à 0.10 g |
| Hydroxystéarate de macrogolglycérol 40 | Agent co-solubilisant / co-gélifiant | 10.0 g |
| Carboxyméthylcellulose de Na | Agent co-solubilisant / co-gélifiant | 0.80 g |
| NaH₂PO₄, H₂O | Agent tampon | 0.520 g |
| Na₂HPO₄, 2H₂O | Agent tampon | 0.652 g |
| PVA | Agent co-solubilisant / co-gélifiant | 0.50 g |
| Eau | - | qs 100 g |

Comme il ressort de la demande et de manière avantageuse, la solution selon l'invention est dépourvue d'agent conservateur de type antimicrobien, en particulier de type ammonium quaternaire, par exemple le chlorure de benzalkonium (BAK).

Dans le cadre de l'invention, par « agent conservateur de type anti-microbien ou anti-microbien », on désigne un agent conservateur présentant des propriétés anti-microbiennes, c'est-à-dire un composé capable de garantir la protection de la solution ophtalmique vis-à-vis d'une éventuelle contamination microbienne. Des composés ammonium quaternaires, en particulier le chlorure de benzalkonium ou le polyquad, éventuellement en association avec l'EDTA, sont généralement utilisés en tant que conservateurs.

D'autres conservateurs sont par exemple :
- les conservateurs à base de guanidine (PHMB, chlorhexidine) ;
- l'alcool benzylique ;
- les parabènes (méthyl, propyl, ...) ;
- les conservateurs à base de mercure, tels que le thimérosal ;
- le chlorobutanol ;
- le chlorure de benzéthonium ;
- les conservateurs oxydants (Purite, ...) ;
- ...

La formulation de l'invention peut être présentée dans des flacons à usage unique (unidose) ou dans un flacon multi-doses du type par exemple Abak® ou Comod® ou équivalent, de tels flacons permettant de délivrer des collyres sans conservateurs pendant plusieurs jours.

L'invention a donc également pour objet un flacon à usage unique (unidose) ou multidose comprenant la solution ophtalmique précédemment décrite, par exemple réalisé en PEBD, avantageusement de qualité EP ne contenant pas d'additifs.

En outre, la solution selon l'invention est stable au moins 24 mois, voire au moins 36 mois, à température ambiante (25°C - 30°C).

Un autre objet de l'invention concerne l'utilisation de cette solution dans le traitement des troubles de la surface oculaire ayant une composante immunitaire inflammatoire.

En ophtalmologie humaine, cette solution est particulièrement adaptée pour le traitement du syndrome de l'oeil sec et/ou de la perte de sensibilité de la cornée.

Des indications relatives à la perte de sensibilité de la cornée sont par exemples celles liées à :
- une opération affectant la cornée ;
- une infection virale (par exemple virus de l'herpès HSV-I, HSV-II ou VZV) ;
- une chirurgie kératoréfractive ou une kératoplastie pénétrante ;
- un patient présentant une kératotomie radiale ;
- une kératotomie photoréfractive ;
- une opération au LASIK (kératomileusis in situ assistée au laser), en particulier LASIK épithélial (EPI-LASIK) ou subépithélial (SB-LASIK).

Par ailleurs, une solution selon l'invention peut être utilisée dans le domaine vétérinaire, notamment dans les cas suivants :
- kératocojonctivite sèche du chien ;
- problèmes de néovascularisation cornéenne, de pigmentation cornéenne et d'infiltration cellulaire dans les kératites superficielles chroniques chez le chien (KSC du chien), le chat et le cheval ;
- infiltration lymphoplasmocytaire de la 3ème paupière chez le chien ;
- kératite éosinophilique du chat et du cheval ;
- kératite ponctuée du chien (Teckel, ...) et du cheval ;
- kératouvéites, endothélites, certains ulcères torpides et fluxion périodique du cheval ;
- en association, dans le traitement des réactions inflammatoires d'origine immune chez le chien, le chat et le cheval ;
- dans la prévention des rejets de greffe cornéenne chez le chien, le chat et le cheval.

En pratique, la solution selon l'invention est administré à l'homme ou à l'animal, par voie topique, à raison d'une à plusieurs gouttes par jour dans chaque oeil.

Selon un autre aspect et en rapport notamment avec l'utilisation d'un dérivé cellulosique, l'invention concerne un procédé de préparation de la solution selon l'invention, comprenant les étapes suivantes :
- pré-solubilisation de l'agent immunomodulateur, à savoir la ciclosporine A, en présence d'au moins une fraction du premier polymère (avantageusement 25% en poids), à savoir un dérivé cellulosique, en présence d'eau et préférentiellement sous agitation ;
- mélange de l'éventuelle fraction restante du premier polymère (avantageusement 75% en poids), à savoir un dérivé cellulosique, avec les deux autres polymères, de l'eau et éventuellement les systèmes tampons et agents isotonisants, avantageusement sous agitation et/ou sous chauffage, par exemple à une température de 60°C ;
- mélange des produits obtenus aux étapes précédentes, avantageusement sous agitation et/ou sous chauffage, par exemple à une température de 60°C.

A l'issue de ce procédé et en vue de sa conservation et de son utilisation en ophtalmologie, une telle solution peut être stérilisée par la chaleur, avantageusement par autoclavage.

Comme il ressort de la présente demande, la solution ophtalmique aqueuse selon l'invention présente plusieurs originalités et avantages :
- elle est stable dans le temps ;
- elle est mieux tolérée par instillation qu'une émulsion huileuse et/ ou une solution hydro-alcoolique;
- elle permet une bonne pénétration dans la cornée, sans accumulation dans les conjonctives, ni passage dans l'humeur aqueuse ou dans le sang ;
- elle renferme un dosage en ciclosporine A adapté au traitement des troubles de la surface oculaire ayant une composante immunitaire inflammatoire, tels que la sécheresse oculaire et/ou la perte de la sensibilité de la cornée.

### EXEMPLES DE REALISATION

L'invention et les avantages qui en découlent ressortiront mieux à la lecture des exemples suivants, donnés à titre indicatif et nullement limitatif, à l'appui des figures annexées, dans lesquelles :
La Figure 1 illustre la pénétration oculaire en fonction du temps, dans les conjonctives (A) et dans la cornée (B), de la ciclosporine A à trois concentrations (0.01, 0.05 et 0.1% respectivement).
La Figure 2 illustre le profil pharmacocinétique, dans la conjonctive (Cj) et dans la cornée (Co), d'une solution selon l'invention à 0.1% en ciclosporine A, après instillation répétée (3 doses par jour pendant 9 jours de (MD), et analyse de la 28ième dose à J10).

### I- Préparation d'une solution à 0,1% (w/w) en ciclosporine A (batch de 100kg)

### I-1/Préparation de la fraction A :

200g de poudre de carboxyméthylcellulose de sodium (correspondant au premier polymère ; 25% de la quantité finale) sont mélangés à 100g de poudre de ciclosporine A (correspondant au principe actif, de qualité conforme à la monographie de la pharmacopée Européenne) puis 3 litres d'eau sont ajoutés. Le tout est mélangé sous agitation, pendant 1 heure.

La ciclosporine A se présente sous la forme d'une poudre pulvérulente, toxique pour le manipulateur. Cette première étape de pré-solubilisation permet l'obtention d'une suspension laiteuse de ciclosporine A, beaucoup plus inoffensive pour le manipulateur.

### 1-2/ Préparation de la fraction B :

500g d'alcool polyvinylique (ou PVA, correspondant au second polymère) sont ajoutés à environ 65 litres d'eau froide (75% du volume final en eau). Le tout est mélangé sous agitation en montant progressivement en température jusqu'à 60°C.

Quand le PVA est solubilisé (après 30 minutes minimum à 300 rpm) sont ajoutés 10kg d'hydroxystéarate de macrogolglycérol 40 (ou PEG 40 hydrogenated castor oil, correspondant au troisième polymère). La solution est mélangée sous agitation pendant environ 20 minutes à 60°C.

Sont ensuite incorporés les agents tampons (NaH₂PO₄, H₂O et Na₂HPO₄, 2H₂O), la solution étant mélangée sous agitation pendant 20 minutes à 60°C. Puis, dans les mêmes conditions (environ 20 min à 60°C), sont ajoutés 600g de carboxyméthylcellulose de sodium (correspondant au premier polymère ; 75% de la quantité finale).

### I-3/ Préparation de la solution finale :

La fraction A est ajoutée à la fraction B. Après homogénéisation, le tout est complété en eau et mélangé pendant 10 minutes à 60°C.

La solution ophtalmique aqueuse ainsi obtenue est stérilisée à 121°C pendant 20 minutes, sous agitation. Elle peut être conditionnée en l'absence de tout conservateur, notamment antimicrobien, soit en flacon à usage unique en PEBD (polyéthylène basse densité) ou en flacon multidoses de type ABAK ou COMOD ou autres.

Il est à noter que le temps de préparation de la solution ophtalmique aqueuse stérile selon l'invention est notablement raccourci (maximum 8 heures) comparé aux protocoles de l'art antérieur, notamment le document WO 2009/099467 dans lequel au moins une étape du mélange se réalise sur une nuit.

Ainsi, ce procédé de fabrication permet une approche conventionnelle de la production industrielle de produits ophtalmiques stériles, sans immobilisation longue de l'outil industriel, facteur de risque en termes de croissance microbienne.

**I-4/ Composition de la solution obtenue :**

| **PRODUIT** | **FONCTION** | **FORMULE CENTESIMALE (g/100g ou %)** |
|---|---|---|
| Ciclosporine A | Principe actif | 0.10 |
| Hydroxystéarate de macrogolglycérol 40 | Agent solubilisant | 10.0 |
| Carboxyméthylcellulose de Na | Agent co-solubilisant / gélifiant | 0.80 |
| NaH₂PO₄, H₂O | Agent tampon | 0.520 |
| Na₂HPO₄, 2H₂O | Agent tampon | 0.652 |
| PVA | Agent gélifiant | 0.50 |
| Eau | Véhicule | qsp 100 |

A noter qu'un protocole identique peut être appliqué pour la préparation d'une solution présentant une concentration en cyclosporine A comprise entre 0.01 et 0,1% (w/v), par exemple égale à 0.05%.

**I-5/ Caractéristiques de la solution obtenue :**

| **Analyses** | **Spécifications** |
|---|---|
| Aspect | Solution légèrement opalescente et incolore |
| pH | 6,79 |
| Osmolalité (mOsmol.kg) | 303 |
| Opalescence (NTU) | 3.65 |
| Coloration (méthode 2.2.2 Ph. Eur. : Une solution est dite *incolore* si elle a l'aspect de l'eau ou du solvant, ou si elle n'est pas plus colorée que la solution témoin B₉) | < B9 |
| Particules (méthode 2.9.19 Ph. Eur.) | Conforme |
| Particules >= 10µm | 570 (<6000) |
| Particules >= 25µm | 17 (<600) |
| Viscosité (mPa.s) | 7,55 |
| Dosage Principe Actif (g/100ml) | 0,099 |

Il apparaît que la solution obtenue présente les avantages suivants :
- un pH neutre ;
- une formulation isotonique ;
- une viscosité proche des larmes humaines. En effet, la viscosité dans les larmes humaines à 20°C serait d'environ 6,5 mPa.s à gradient de vitesse proche de zéro. Or, la force exercée par les paupières lors d'un clignement normale est égale à environ 0,2N et lors d'un clignement forcé environ 0,7 - 0,8N. Ainsi, en présence de la solution selon l'invention, le patient n'éprouve pas de sensation de gêne et d'irritation, qui apparaît lorsque les paupières doivent exercer, lors d'un clignement, une force supérieure ou égale à 0,9N, cette sensation étant provoquée par les clignements reflexes qui induisent une élimination rapide grâce aux larmes.

Ainsi, cette solution constitue une formulation haute tolérance, obtenue malgré la très faible solubilité du principe actif en présence dans l'eau.

### II- Stabilité de la solution selon l'invention

Des analyses de stabilité à 25°C (Tableau 1) et à 40°C (Tableau 2) ont démontré une très bonne stabilité de la solution selon l'invention, ce qui permet de revendiquer une durée de péremption de 36 mois dans des conditions de stockage à température ambiante.

**Tableau 1 : Stabilité de la solution ophtalmique aqueuse selon l'invention à 25°C et 40% d'humidité relative**

| **ANALYSES** | **NORMES** | **Initiale** | **6 mois** | **12 mois** | **24 mois** | **36 mois** |
|---|---|---|---|---|---|---|
| **Aspect** | Solution visqueuse, opalescente et incolore à légèrement jaune | conforme | conforme | conforme | conforme | conforme |
| **pH** | 6,5-7,0 | 6,79 | 6,72 | 6,70 | 6,71 | 6,58 |
| **Osmolarité (mosmol/kg)** | 290-350 | 303 | 300 | 303 | 296 | 301 |
| **Opalescence (NTU)** | <5,1 NTU (sol III Ph. Eur.) | 3,65 | 4,33 | 4,03 | 4,37 | 4,81 |
| **Coloration** | <B9 | <B9 | <B9 | <B9 | <B9 | <B9 |
| **Particules** | Absence | Absence | Absence | Absence | Absence | Absence |
| **Viscosité (mPa.s)** | >5mPa.s | 7,55 | 7,55 | 7,51 | 7,17 | 7,25 |
| **Teneur en ciclosporine A** | 0,090%-0,110% | 0,099 | 0,100 | 0,100 | 0,099 | 0,100 |
| **Teneur totale en impuretés** | <2,5% | 1,15 | 1,22 | 1,03 | 0,92 | 1,14 |
| **Contrôle de stérilité** | Conforme | Conforme | Conforme | Conforme | Conforme | Conforme |

**Tableau 2 : Stabilité de la solution ophtalmique aqueuse selon l'invention à 40°C et 25% d'humidité relative**

| **ANALYSES** | **NORMES** | **Initial** | **3 mois** | **6 mois** | **12 mois** |
|---|---|---|---|---|---|
| **Aspect** | Solution visqueuse, opalescente et incolore à légèrement jaune | conforme | conforme | conforme | conforme |
| **Osmolarité (mosmol/kg)** | 290-350 | 303 | 304 | 311 | 318 |
| **Opalescence (NTU)** | <5,1 NTU (sol III Ph. Eur.) | 3,65 | 4,04 | 4,60 | 4,62 |
| **Coloration** | <B9 | <B9 | <B9 | <B9 | <B9 |
| **Particules** | Absence | Absence | Absence | Absence | Absence |
| **Viscosité (mPa.s)** | >5mPa.s | 7,55 | 7,17 | 7,08 | 6,66 |
| **Teneur en ciclosporine A** | 0,0900%-0,1100% | 0,0994 | 0,0991 | 0,0992 | 0,0981 |
| **Teneur totale en impuretés** | <2,5% | 1,15 | 1,12 | 1,23 | 1,04 |

### III- Tolérance oculaire de la solution selon l'invention

Une solution à 0,1% en poids de ciclosporine A, préparée comme décrit ci-dessus, a été testée chez le lapin, à raison de 8 instillations quotidiennes de 50µl pendant 7 jours.

Les examens oculaires suivants ont été réalisés :
- observations oculaires à l'aide de l'échelle de Draize, sur les deux yeux en utilisant un ophtalmoscope, avant le traitement et après la dernière instillation du jour ;
- examens oculaires à l'aide de l'échelle de Mc Donald-Shadduck, sur les deux yeux en utilisant une lampe à fente, avant le traitement, au jour 1 juste avnat la dernière administration puis au jour 8 ;
- prélèvement et fixation de tous les yeux.

Il a été observé une bonne tolérance oculaire chez le lapin de la solution selon l'invention, contrairement aux formulations, notamment par rapport à une préparation alcoolique avec un tensioactif non ionique tel que le MODUSIK-A®.

### IV- Etude cinétique d'une solution selon l'invention par rapport à une émulsion commerciale (Restasis™)

La distribution oculaire de la ciclosporine A a été analysée sur un pool de conjonctives (bulbaires et palpébrales) et sur la cornée, suite à une instillation unique dans l'oeil droit d'un lapin de 50 µl :
- d'une solution aqueuse selon l'invention, contenant 0.05% (en poids) de ciclosporine A, préparée comme décrit au point I et notée T1580 ;
- du produit Restasis™, une émulsion commerciale comprenant 0.05% (en poids) de ciclosporine A.

Pour ce faire, 48 lapins pigmentés (fauve de Bourgogne - Ref: HY R NZ 104) ont été divisés en 2 groupes de 24 animaux. Chaque sous-groupe a ensuite été subdivisé en 6 groupes de 4 animaux, correspondant à 6 points d'analyse (20 minutes, 40 minutes, 1 heure, 4 heures, 12 heures et 24 heures, respectivement). Les animaux ont reçu une instillation unique de 50 µl de T1580 ou de Restasis™ dans l'oeil droit. A chaque temps d'analyse, les animaux ont été euthanasiés pour échantillonner les conjonctives (bulbaires et palpébrales) (Cj) et la cornée (Co), conservées à -20°C jusqu'au dosage. La teneur en ciclosporine A a été déterminée dans les conjonctives et la cornée par HPLC-MS (Chromatographie Liquide Haute Performance, couplée à la Spectrométrie de Masse).

Les résultats sont présentés dans le Tableau 3 ci-dessous, dans lequel :
- Cₘₐₓ correspond à la concentration maximale observée ;
- Tₘₐₓ représente le temps nécessaire pour que la concentration maximale soit atteinte ; et
- AUC (« Area Under the curve ») est l'aire sous la courbe en fonction du temps comprise entre le temps zéro et le temps correspondant à la dernière concentration mesurable. On utilise pour ce calcul, la méthode aléatoire pour échantillon non séquentiel.

**Tableau 3 : Comparaison pharmacocinétique de la solution ophtalmique aqueuse selon l'invention et de l'émulsion commerciale Restasis™, contenant chacune 0,05% de ciclosporine A.**

| | **Ciclosporine A dans les conjonctives (Cj)** | | | **Ciclosporine A dans la cornée (Co)** | | |
|---|---|---|---|---|---|---|
| | **Cₘₐₓ (ng/g)** | **Tₘₐₓ (h)** | **AUC** | **Cₘₐₓ (ng/g)** | **Tₘₐₓ (h)** | **AUC** |
| **T1580** | 453 | 0,33 | 966 | 769 | 0,66 | 8189 |
| **Restasis™** | 257 | 0,33 | 924 | 504 | 1 | 6516 |

Il ressort que suite à une instillation de 50µl de T1580 ou de Restasis™ dans l'oeil droit de lapins pigmentés, la ciclosporine A est retrouvée principalement dans les conjonctives entre 20 minutes et 4 heures, et dans la cornée entre 20 minutes et 24 heures. Les animaux traités avec T1580 présentent une quantité supérieure de ciclosporine A, entre 20 minutes et 12 heures, dans les conjonctives et la cornée, en particulier au niveau de la cornée, comparativement aux animaux traités avec Restasis™. Ceci met en évidence une meilleure disponibilité du principe actif formulé selon l'invention.

### V- Etude pharmacocinétique d'une solution selon l'invention administrée en instillation mono-dose, à trois concentrations distinctes

La pénétration oculaire d'une monodose de solution selon l'invention a été analysée dans les conjonctives et la cornée, après instillation de 25µl dans chaque oeil de lapins pigmentés, en fonction du temps (0.33, 0.66, 1, 2, 4, 8, 12 et 24h après instillation). Trois concentrations de ciclosporine A ont été testées : 0.01, 0.05 et 0.1% w/w. L'étude a porté sur 72 lapins, soit 3 lapins par temps.

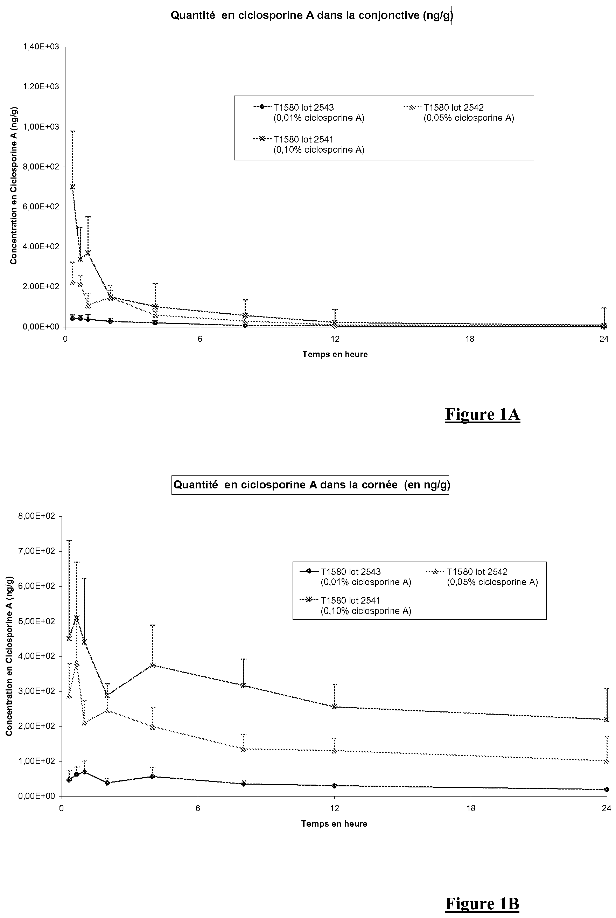

La Figure 1A, relative aux conjonctives, et la figure 1B, relative à la cornée, montrent un effet dose-dépendant entre la concentration en ciclosporine A et le profil pharmacocinétique obtenu pour les conjonctives et la cornée.

On note que l'AUC obtenue pour la cornée représente 4 à 5 fois celle mesurée pour les conjonctives, et que la ciclosporine A réside plus longtemps dans la cornée que dans les conjonctives, ce qui démontre un tropisme particulier de la ciclosporine A pour la cornée.

### VI- Etude pharmacocinétique d'une solution selon l'invention administrée en instillations répétées

Dans cette étude, des lapins pigmentés ont subi une instillation dans chaque oeil (oeil droit et gauche) d'une dose (25 µl) d'une solution selon l'invention contenant 0.1% (w/w) de ciclosporine A, à raison de trois fois par jour pendant 10 jours.

Plus précisément, l'instillation se poursuit pendant 9 jours (soit 27 instillations) puis le profil pharmacocinétique est déterminé à la 28^{ème} instillation, soit au jour 10 (D10), le matin. Un échantillonnage est réalisé au temps 0 (C0 estimation « trough ») puis à 0.33h et 0.66h correspondant au Cmax pour les conjonctives et la cornée, puis à 1, 2, 4, 8 et 24 heures.

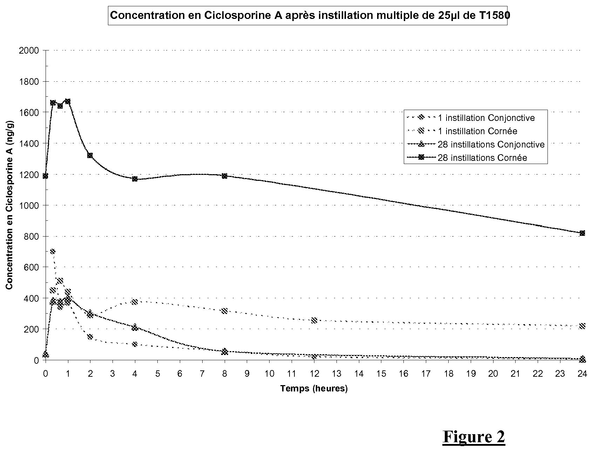

Les résultats sont présentés à la Figure 2. A cette concentration et à cette posologie :
- il n'y a pas d'accumulation de la ciclosporine A dans les conjonctives, ni de persistance de la concentration. Dans cette structure, la couverture immunomodulatrice sur le nycthémère semble donc limitée dans le temps ;
- il y a accumulation dans la cornée et une dose de l'ordre de 1 000 à 2 000 ng de ciclosporine A /g de cornée persiste pendant 24 heures dans la cornée. Dans la cornée, la couverture immunomodulatrice nycthémérale est donc satisfaisante.
Par ailleurs, il a été observé l'absence de passage dans l'humeur aqueuse et un passage dans le sang insignifiant.

En conclusion, l'efficacité de la solution selon l'invention est démontrée puisque les valeurs obtenues sont du même ordre de grandeur que les données partielles disponibles pour le Restasis™ et sont compatibles avec celles de la littérature qui préconise une concentration en actif, dans les tissus cibles, de l'ordre de 100 à 400 ng/g pour obtenir un effet immunomodulateur.

## Revendications

1. Solution ophtalmique aqueuse comprenant :
- la ciclosporine A en tant qu'agent immunosuppresseur;
- un dérivé cellulosique en tant que premier polymère ;
- un dérivé polyvinylique en tant que deuxième polymère ;
- un hydroxystéarate de macrogolglycérol en tant que troisième polymère.

2. Solution selon la revendication 1, ***caractérisée* en ce que** la ciclosporine A représente au moins 0,05% en poids de la solution, avantageusement entre 0,05 et 0,1%.

3. Solution selon l'une des revendications précédentes, ***caractérisée* en ce qu'**elle est dépourvue de chlorure de benzalkonium (BAK).

4. Solution selon l'une des revendications précédentes, ***caractérisée* en ce qu'**elle présente une viscosité Brookfield à 25°C inférieure à 50 mPa.s, avantageusement comprise entre 5 et 15 mPa.s.

5. Solution selon l'une des revendications précédentes, ***caractérisée* en ce que** le dérivé cellulosique est un éther de cellulose, avantageusement choisi parmi la méthylcellulose, l'éthylcellulose, l'hydroxyéthylcellulose, l'hydroxypropylcellulose, la carboxymethylcellulose, l'hydroxypropylméthylcellulose ou un de leurs sels, encore plus avantageusement de la carboxyméthylcellulose de sodium.

6. Solution selon l'une des revendications précédentes, ***caractérisée* en ce que** le dérivé polyvinylique est de l'alcool polyvinylique.

7. Solution selon l'une des revendications précédentes, ***caractérisée* en ce que** l'hydroxystéarate de macrogolglycérol est l'hydroxystéarate de macrogolglycérol 40.

8. Solution selon l'une des revendications précédentes, ***caractérisée* en ce que** le dérivé cellulosique représente entre 0,1 et 3% en poids de la solution, avantageusement entre 0,5 et 1,5%, encore plus avantageusement 0,8%.

9. Solution selon l'une des revendications précédentes, ***caractérisée* en ce que** le dérivé polyvinylique représente entre 0,25 et 3% en poids de la solution, avantageusement 0,5%.

10. Solution selon l'une des revendications précédentes, ***caractérisée* en ce que** l'hydroxystéarate de macrogolglycérol représente entre 0,5% et 20% en poids de la solution, avantageusement entre 5 et 15%, encore plus avantageusement 10%.

11. Flacon à usage unique ou multidose réalisé en PEBD comprenant la solution objet de l'une des revendications 1 à 10.

12. Solution selon l'une des revendications 1 à 10 pour son utilisation dans le traitement des troubles de la surface oculaire ayant une composante immunitaire inflammatoire, avantageusement du syndrome de l'oeil sec et/ou de la perte de la sensibilité de la cornée.

13. Solution pour son utilisation selon la revendication 12 ***caractérisée* en ce qu'**une à plusieurs gouttes par jour de ladite solution sont administrées à l'homme ou à l'animal, par voie topique, dans chaque oeil.

14. Procédé de préparation d'une solution selon l'une des revendications 1 à 10 comprenant les étapes suivantes :
- solubilisation de l'agent immunosuppresseur en présence d'au moins une fraction du premier polymère, en présence d'eau et préférentiellement sous agitation ;
- mélange de l'éventuelle fraction restante du premier polymère avec les deux autres polymères, de l'eau et éventuellement les systèmes tampons et agents isotonisants, avantageusement sous agitation et/ou sous chauffage, par exemple à une température de 60°C ;
- mélange des produits obtenus aux étapes précédentes, avantageusement sous agitation et/ou sous chauffage, par exemple à une température de 60°C.

## Patentansprüche

1. Wässrige ophthalmische Lösung, umfassend
- Ciclosporin A als Immunsuppressivum
- ein Cellulosederivat als erstes Polymer;
- ein Polyvinylderivat als zweites Polymer;
- ein Makrogolglycerinhydroxystearat als drittes Polymer.

2. Lösung nach Anspruch 1, ***dadurch gekennzeichnet, dass*** das Ciclosporin A mindestens 0,05% Gewichts-% der Zusammensetzung umfasst, am besten zwischen 0,05 und 0,1%.

3. Lösung nach einem der vorhergehenden Ansprüche, ***dadurch gekennzeichnet, dass*** sie kein Benzalkoniumchlorid (BAK) enthält.

4. Lösung nach einem der vorhergehenden Ansprüche, ***dadurch gekennzeichnet, dass*** sie eine Brookfield-Viskosität bei 25°C von unter 50 mPa.s aufweist, am besten zwischen 5 und 15 mPa.s.

5. Lösung nach einem der vorhergehenden Ansprüche, ***dadurch gekennzeichnet, dass*** das Cellulosederivat ein Celluloseäther ist, vorteilhafterweise ausgewählt aus Methylcellulose, Ethylcellulose, Hydroxyethylcellulose, Hydroxypropylcellulose, Carboxymethylcellulose, Hydroxypropylmethylcellulose oder eines ihrer Salze, noch vorteilhafter ist Natrium- Carboxymethylcellulose.

6. Lösung nach einem der vorhergehenden Ansprüche, ***dadurch gekennzeichnet, dass*** das Polyvinylderivat ein Polyvinylalkohol ist.

7. Lösung nach einem der vorhergehenden Ansprüche, ***dadurch gekennzeichnet, dass*** das Macrogolglycerol-hydroxystearat 40 ist.

8. Lösung nach einem der vorhergehenden Ansprüche, ***dadurch gekennzeichnet, dass*** das Cellulosederivat zwischen 0,1 und 3% Gewichts-% der Lösung, vorteilhafterweise zwischen 0,5 und 1,5% Gewichts-% und noch besser 0,8% darstellt.

9. Lösung nach einem der vorhergehenden Ansprüche, ***dadurch gekennzeichnet, dass*** das Cellulosederivat zwischen 0,25 und 3% Gewichts-% der Lösung, vorteilhafterweise 0,5 Gewichts-% darstellt.

10. Lösung nach einem der vorhergehenden Ansprüche, ***dadurch gekennzeichnet, dass*** das Macrogolglycerol-hydroxystearat zwischen 0,5 und 20% Gewichts-% der Lösung, vorteilhafterweise zwischen 5 und 15% Gewichts-% und noch besser 10 % darstellt.

11. Flakon zum Einmal- oder Mehrfachgebrauch aus PEBD, der die Lösung, die Gegenstand eines der Ansprüche 1 bis 10 ist, enthält.

12. Lösung nach einem der vorhergehenden Ansprüche 1 bis 10 zur Verwendung in der Behandlung von Problemen der Augenoberfläche mit einer entzündlichen Immunkomponente, am besten das Trockene-Augen-Syndrom und/oder Verlust der Hornhautsensibilität.

13. Lösung zur Verwendung nach Anspruch 12 ***dadurch gekennzeichnet, dass*** einer oder mehrere Tropfen dieser Lösung pro Tag Menschen oder Tieren auf topischem Weg, in jedes Auge verabreicht werden.

14. Verfahren zur Herstellung einer Lösung nach irgendeinem der Ansprüche 1 bis 10, das die folgenden Schritte umfasst:
- Solubilisierung des Immunsuppressivums in Anwesenheit mindestens einer Fraktion des ersten Polymers, in Anwesenheit von Wasser und vorzugsweise unter Schütteln;
- Mischung der eventuell verbleibenden Fraktion des ersten Polymers mit den beiden anderen Polymeren, Wasser und eventuell Puffersystemen und isotonisierenden Wirkstoffen, vorzugsweise unter Schütteln und/oder unter Erwärmung, zum Beispiel auf eine Temperatur von 60°C;
- Mischen der in den vorangegangenen Schritten erhaltenen Produkte, vorzugsweise unter Schütteln und/ oder unter Erwärmung, zum Beispiel auf eine Temperatur von 60°C.

## Claims

1. An aqueous ophthalmic solution comprising :
- ciclosporin A as an immunosuppressive agent;
- a cellulose derivative as a first polymer ;
- a polyvinyl derivative as a second polymer ;
- a macrogolglycerol hydroxystearate as a third polymer.

2. The solution according to claim 1, **characterized in that** ciclosporin A represents at least 0.05% by weight of the solution, preferably between 0.05 and 0.1%.

3. The solution according to one of the preceding claims, **characterized in that** it is free of benzalkonium chloride (BAK).

4. The solution according to one of the preceding claims, **characterized in that** it has a Brookfield viscosity at 25°C of less than 50 mPa.s, advantageously between 5 and 15 mPa.s.

5. The solution according to one of the preceding claims, **characterized in that** the cellulose derivative is a cellulose ether, preferably selected from methylcellulose, ethylcellulose, hydroxyethylcellulose, hydroxypropylcellulose, carboxymethylcellulose, hydroxypropylmethylcellulose or a salt thereof, more preferably sodium carboxymethylcellulose.

6. The solution according to one of the preceding claims, **characterized in that** the polyvinyl derivative is polyvinyl alcohol.

7. The solution according to one of the preceding claims, **characterized in that** the macrogolglycerol hydroxystearate is macrogolglycerol hydroxystearate 40.

8. The solution according to one of the preceding claims, **characterized in that** the cellulose derivative represents between 0.1 and 3% by weight of the solution, preferably between 0.5 and 1.5%, even more advantageously 0.8%.

9. The solution according to one of the preceding claims, **characterized in that** the polyvinyl derivative represents between 0.25 and 3% by weight of the solution, preferably 0.5%.

10. The solution according to one of the preceding claims, **characterized in that** the macrogolglycerol hydroxystearate represents between 0.5% and 20% by weight of the solution, preferably between 5 and 15%, even more advantageously 10%.

11. Disposable or multidose vial made of LDPE comprising the solution of one of claims 1 to 10.

12. The solution according to one of claims 1 to 10 for its use in the treatment of ocular surface disorders having an inflammatory immune component, preferably dry eye syndrome and / or loss of sensitivity of the cornea.

13. The solution for its use according to claim 12 **characterized in that** one to several drops per day of said solution are administered to humans or animals, topically, in each eye.

14. Process for preparing a solution according to one of claims 1 to 10 comprising the following steps:
- solubilization of the immunosuppressive agent in the presence of at least a fraction of the first polymer, in the presence of water and preferably with stirring;
- mixture of the possible remaining fraction of the first polymer with the other two polymers, water and optionally the buffer systems and isotonizing agents, advantageously with stirring and/or under heating, for example at a temperature of 60°C;
- mixing the products obtained in the preceding steps, advantageously with stirring and / or under heating, for example at a temperature of 60°C.
